# EUROPÄISCHE PATENTSCHRIFT

(11) **EP 0 360 284 B1**
(45) Veröffentlichungstag und Bekanntmachung des Hinweises auf die Patenterteilung: **12.05.1993**
(21) Anmeldenummer: 89117568.9
(22) Anmeldetag: 22.09.1989
(51) Int. Cl.: A61L 2/18, A61K 6/10

(54) **Verfahren zur Behandlung von Kieferabdrücken und dafür geeignete wässrige Desinfektionsmittellösungen**
Process for the treatment of oral impressions and aqueous disinfectant solutions useful for this purpose
Procédé pour le traitement d'impressions maxillaires et solutions aqueuses de désinfectants utilisables dans ce procédé

(30) Priorität: 23.09.1988 DE 3832417
(43) Veröffentlichungstag der Anmeldung: 28.03.1990
(73) Patentinhaber: THERA Patent GmbH & Co. KG Gesellschaft für industrielle Schutzrechte, D-82229 Seefeld (DE)
(72) Erfinder: Gasser, Oswald, Dr., D-8031 Seefeld (DE); Ellrich, Klaus, Dr., D-8031 Wörthsee (DE)
(74) Vertreter: Abitz, Walter, Dr.-Ing.

(56) Entgegenhaltungen:
- EP-A- 0 273 450
- DE-A- 1 466 968
- DE-A- 3 410 923
- CHEMICAL ABSTRACTS, Band 109, Nr. 8, 22. August 1988, Seite 435, Zusammenfassung Nr. 61410z, Columbus, Ohio, US; J. VIOHL et al.: "Dimensional stability of alginate impressions. Dimensional stability of alginate impressions and hardness of the gypsum casts during disinfection", & DTSCH. ZAHNAERZTL. Z. 1988, 43(4), 477-81
- R. PHILLIPS: "Skinner's Science of Dental Materials", 1982, Ausgabe 8, Seite 128, W.B. Saunders Co., Philadelphia, US

## Beschreibung

Die Herstellung von Zahnersatz im Dentallaboratorium erfolgt üblicherweise über ein Arbeitsmodell, welches die Zahn- und Kieferverhältnisse des Patienten so originalgetreu wie möglich wiedergibt. Hierbei wird vom behandelnden Zahnarzt mittels elastischer Abformmaterialien, die sich durch hohe Abformgenauigkeit, hohe Formbeständigkeit und gute Detailwiedergabe auszeichnen, zunächst eine negative Abformung vorgenommen. Solche Abformmaterialien sind beispielsweise die sogenannten reversiblen Hydrokolloide auf Basis Agar-Agar, irreversible Hydrokolloide auf Alginatbasis, Polysulfide, Polyether, und kondensations- und additionsvernetzende Silikone.

Die so erhaltenen Kieferabdrücke, oder auch Negativformen genannt, können anschließend vom Zahnarzt oder Zahntechniker mit einem Modellmaterial ausgegossen werden und führen so zum Arbeitsmodell. Als Modellmaterialien stehen insbesondere Gipse zur Verfügung, die sich aber hinsichtlich Qualität und Preis stark unterscheiden können. Hierbei ist es naturgemäß so, daß die qualitativ hochwertigen Gipse, die sich auch durch eine hohe Oberflächengüte auszeichnen, vergleichsweise teuer sind.

Man hat deswegen bereits versucht, aus billigeren Gipsarten hergestellte Modelle durch Oberflächenbehandlungen zu vergüten. Dies kann beispielsweise durch Auftragen eines härtbaren Lackes geschehen. Ein befriedigender Lösungsweg ist dies in der Regel aber nicht, da sich die Härtungsschicht nur schlecht mit dem Gips verbinden läßt und zudem die Genauigkeit des Modellmaterials durch die aufgetragene Schicht beeinflußt wird.

Ein weiteres Problem, das die Oberflächengüte solcher Gipse betrifft, ist der negative Einfluß von oberflächenaktiven Substanzen, die sich auf der Oberfläche der Negativformen befinden können. Der Einsatz solcher oberflächenaktiver Substanzen oder Tenside ist immer dann notwendig, wenn die Negativform gründlich gereinigt, desinfiziert oder oberflächenentspannt werden soll. Die oberflächenaktiven Substanzen reichern sich dann an der Oberfläche der Negativformen aus beispielsweise Hydrokolloiden an und können trotz Abspülens nicht vollständig entfernt werden. Dies führt letztlich zu einer Beeinträchtigung der Oberflächengüte des Gipses und damit zu einer Beeinträchtigung der Abformgenauigkeit des Gipsmodells.

Aus "The Science of Dental Materials", 5. Aufl., 1960, W. B. Saunders Company, Philadelphia und London, ist bekannt, den Abformmaterialien u.a. Fluorverbindungen zuzusetzen, um die Oberflächenhärte der mit ihrer Hilfe hergestellten Gipsmodelle zu härten (s. Seite 94). Der schädliche Einfluß der an den Abdrücken haftenden oberflächenaktiven Substanzen wird dadurch jedoch nicht berührt. Auch unter Verwendung von Fluorverbindungen enthaltenden Abformmaterialien werden bei Einsatz billigerer Gipsarten letztlich Gipsmodelle erhalten, deren Oberflächen sandig sind und deren Konturschärfe zu wünschen übrig läßt.

Aufgabe der Erfindung ist es daher, einen Weg zu finden, wie der schädliche Einfluß von auf den Negativformen haftenden oberflächenaktiven Substanzen auf die Oberflächenbeschaffenheit der Gipse verhindert werden kann.

Gelöst wird diese Aufgabe dadurch, daß die Oberfläche der Kieferabdrücke oder Negativformen mit einer wässrigen Lösung behandelt wird, die 0,01 bis 10 Gew.-% einer Fluorverbindung enthält. Vorzugsweise beträgt die Menge an Fluorverbindung innerhalb der Lösung 0,1 bis 5 Gew.-% und insbesondere 0,5 bis 2 Gew.-%.

Als Fluorverbindungen kommen anorganische Fluorverbindungen, deren Löslichkeiten innerhalb der genannten Konzentrationsbereiche liegen, wie Metallfluoride, z.B. die Alkalifluoride, Magnesiumfluorid, Zinkfluorid oder Zinnfluoride, oder komplexe Fluorverbindungen, die Metalle der dritten bis fünften Hauptgruppe sowie Metalle der Nebengruppen und Seltenen Erden des chemischen Periodensystems enthalten, in Betracht. Bevorzugt sind Natriumzinkfluorid und inbesondere komplexe Fluorverbindungen der Formel A₂MF₆, wobei A vorzugsweise ein Alkalimetall, insbesondere Natrium oder Kalium, und M Silicium, Zinn, Titan, Zirkonium oder Hafnium bedeuten. Besonders geeignete komplexe Fluorverbindungen sind K₂TiF₆ und K₂ZrF₆.

Die Konzentration der Fluorverbindung in der Lösung hängt vom Einsatzzweck, der Löslichkeit und von der Stabilität der Fluorverbindung ab und kann im einzelnen anhand von wenigen Versuchen optimiert werden. Bei Einsatz von komplexen Fluorverbindungen, wie beispielsweise K₂TiF₆ und K₂ZrF₆, haben sich als besonders günstig Konzentrationsbereich von 0,5 bis 2 Gew.-% erwiesen.

Die erfindungsgemäß anzuwendenden Lösungen können weiterhin solche Zusatzstoffe enthalten, die sie zur gleichzeitigen Verwendung als Reinigungslösung, Desinfektionslösung oder Entspannungsspray geeignet machen. So sind beispielsweise Desinfektionsmittel wie quaternäre Ammonium-Verbindungen, organische Persäuren, z.B. Peressigsäure, Aldehyde, beispielsweise Glutaraldehyd und Glyoxal, Phenole und Alkohole und andere bekannte Desinfektionsmittel geeignete Zusatzstoffe. Weiterhin können geringe Mengen an oberflächenaktiven Substanzen vorhanden sein, beispielsweise Tenside aus der Gruppe der nicht ionischen und anionischen Tenside. Weiterhin können der Lösung Duft- und Geschmacksstoffe, Farbstoffe sowie gegebenenfalls Indikatoren zugesetzt werden.

Bei der Durchführung des erfindungsgemäßen Verfahrens können die Lösungen beispielsweise in Form von Sprays, enthaltend mindestens eine der genannten Fluorverbindungen, aufgesprüht werden. Zweckmäßig ist auch die Verwendung von Tauchbädern, die eine Fluorverbindung im oben genannten Konzentrationsbereich enthalten.

Bei der Verwendung von Tauchbädern wird die Negativform vorteilhaft über einen kurzen Zeitraum in die Lösung eingetaucht. Brauchbare Tauchzeiten reichen von 5 Sekunden bis 30 Minuten. Die Tauchzeit hängt aber stark von der beabsichtigten Wirkung ab. Soll gleichzeitig gereinigt und desinfiziert werden, so bieten sich Tauchzeiten von 1 bis 20, insbesondere 5 bis 15 Minuten an. Um die Dimensionsstabilität, vor allem der irreversiblen Hydrokolloid-Negativformen, zu gewährleisten, ist es sinnvoll, daß neben dem Desinfektionsmittel, z.B. ein Gemisch aus Glutaraldehyd und Glyoxal, oder geringe Mengen, z.B. 1 bis 10 Gew.-%, an Alkoholen vorhanden sind, damit Negativformen aus hydrophilen Abdruckmaterialien bei der Desinfektion keiner Quellung oder Schrumpfung unterliegen.

Die Herstellung der erfindungsgemäß zu verwendenden Lösungen erfolgt in einfacher Weise durch Auflösung der für die gewünschten Konzentrationen passenden Menge an Fluorverbindung in Wasser und gegebenenfalls Alkohol. So kann als Tauch- bzw. Sprühlösung zur Behandlung von Negativformen eine 0,01 bis 10 Gew.-% Fluorverbindung enthaltende wässrige oder wässrig/alkoholische Lösung verwendet werden. Bei Durchführung des erfindungsgemäßen Verfahrens im Rahmen der Verwendung einer Desinfektionsmittellösung wird die Fluorverbindung in der gewünschten Konzentration in einem wässrigen oder wässrig/alkoholischen Desinfektionsmittelgemisch aufgelöst. Hier kann es gegebenenfalls vorteilhaft sein, zur Steigerung der Desinfektionsmittelwirkung gleichzeitig Tenside einzusetzen. Durch die erfindungsgemäß zu verwendende Fluorverbindung wird der schädigende Einfluß der Tenside auf die Oberfläche der unter Verwendung der Negativformen hergestellten Gipsmodelle beseitigt.

### Beispiel 1

### Herstellung einer Tauchlösung.

0,5 Gew.-Teile K₂TiF₆ werden in 99,5 Gew.-Teilen H₂O dest. so lange gerührt bis eine homogene klare Lösung entsteht.

### Beispiel 2

Ein von einer Testperson unter Verwendung eines Alginatabformmaterials, erhalten aus Vermischung von 40 Gewichtsteilen Leitungswasser und 20 Gewichtsteilen Alginatabdruckpulver, bestehend aus 11 % Kaliumalginat, 19,5 % Calciumsulfat, 3,7 % Tetranatriumdiphosphat, 2,8 % Kaliumtitanfluorid, 60 % Kieselgur, 1 % pyrogenes Siliciumdioxid und 2 % Zinkoxid, abgenommener Oberkieferabdruck (Negativform) wird für einen Zeitraum von 10 Sekunden vollständig in ein Tauchbad, bestehend aus 500 ml Lösung von Beispiel 1, eingetaucht. Zum Vergleich wird aus dem gleichen Abformmaterial ein zweiter Abdruck vom gleichen Patienten genommen, aber nicht getaucht. Beide Abdrücke werden anschließend mit einem Dentalgips (Moldano, Fa. Bayer) ausgegossen. Der Gips wird für 30 Minuten in dem Abdruck belassen und anschließend vorsichtig aus dem Alginatabdruck entfernt. Bei dem aus dem nicht getauchten Abdruck entnommenen Gipsmodell (Vergleichsversuch) wird eine relativ rauhe, leicht sandig wirkende Oberfläche erhalten, während auf dem Alginatabdruck eine dünne Schicht des blauen, nicht abgebundenen Gipsmaterials zurückbleibt. Bei dem aus dem getauchten Abdruck entnommenen Gipsmodell erhält man eine glatte, harte Oberfläche und der zurückbleibende Abdruck zeigt keinerlei Reste von nicht abgebundenem Gips.

### Beispiel 3

### Herstellung einer Desinfektionsmittellösung.

99,5 Gewichtsteile eines Desinfektionsmittels, enthaltend 0,9 Gew.-% Glutaraldehyd, 1,8 Gew.-% Glyoxal, 4,5 Gew.-% Ethanol und Rest Wasser, werden mit 0,5 Gewichtsteilen Natriumfluorid so lange gerührt, bis eine homogene klare Lösung entsteht.

### Beispiel 4

### Herstellung einer Desinfektionsmittellösung.

99,5 Gewichtsteile eines Desinfektionsmittels, enthaltend 0,9 Gew.-% Glutaraldehyd, 1,8 Gew.-% Glyoxal, 4,5 Gew.-% Ethanol und Rest Wasser, werden mit 0,5 Gewichtsteilen K₂TiF₆ so lange gerührt, bis eine homogene klare Lösung entsteht.

### Beispiel 5

Analog der Vorgehensweise von Beispiel 2 werden Alginatabdrücke von Testpersonen genommen und jeweils für einen Zeitraum von 10 Minuten vollständig in ein Tauchbad, bestehend aus je 500 ml Lösung von Beispiel 3 und 4, eingetaucht. Zum Vergleich wird aus dem gleichen Abformmaterial je ein zweiter Abdruck vom gleichen Patienten genommen und in eine Lösung getaucht, die den Lösungen der Beispiele 3 und 4 entspricht, jedoch den erfindungsgemäßen Zusatz einer Fluorverbindung nicht enthält. Anschließend wird wie in Beispiel 2 mit Dentalgips (Moldano, Fa. Bayer) ausgegossen. Das unter Verwendung des erfindungsgemäß behandelten Abdruckes hergestellte Gipsmodell weist eine glatte, harte Oberfläche auf. Auf dem zurückbleibenden Alginatabdruck ist keine Gipsschicht zu sehen. Bei dem Gipsmodell, das unter Verwendung des nicht erfindungsgemäß behandelten Abdruckes erhalten worden ist, ist eine relativ rauhe, leicht sandig wirkende Oberfläche festzustellen. Auf dem zuürckbleibenden Alginatabdruck findet man eine dünne Schicht des blauen, nicht abgebundenen Gipsmaterials vor.

## Patentansprüche

1. Verfahren zur Behandlung von aus üblichen Abformmaterial bestehenden Kieferabdrücken, dadurch gekennzeichnet, daß die Oberfläche der Abdrücke vor dem Ausgießen mit Gips mit einer wässrigen Lösung behandelt wird, die 0,01 bis 10 Gew.-% einer Fluorverbindung enthält.

2. Verfahren nach Anspruch 1, dadurch gekennzeichnet, daß die Lösung 0,1 bis 5 Gew.-%, vorzugsweise 0,5 bis 2 Gew.-%, einer Fluorverbindung enthält.

3. Verfahren nach den Ansprüchen 1 oder 2, dadurch gekennzeichnet, daß als Fluorverbindung ein Metallfluorid, vorzugsweise aus der Gruppe bestehend aus den Alkalimetallfluoriden, Magnesiumfluorid, Zinkfluorid und Zinnfluoriden, verwendet wird.

4. Verfahren nach den Ansprüchen 1 oder 2, dadurch gekennzeichnet, daß als Fluorverbindung eine komplexe Fluorverbindung verwendet wird.

5. Verfahren nach Anspruch 4, dadurch gekennzeichnet, daß eine Verbindung der Formel A₂MF₆ verwendet wird, worin A ein Alkalimetall, vorzugsweise Natrium oder Kalium, und M Titan, Zirkonium oder Hafnium bedeuten.

6. Verfahren nach Anspruch 5, dadurch gekennzeichnet, daß K₂TiF₆ oder K₂ZrF₆ verwendet werden.

7. Verfahren nach den Ansprüchen 1 bis 6, dadurch gekennzeichnet, daß die Lösung weiterhin übliche Zusatzstoffe enthält.

8. Verfahren nach Anspruch 7, dadurch gekennzeichnet, daß die Behandlung durch Besprühen oder Tauchen der Kieferabdrücke erfolgt.

9. Verfahren nach den Ansprüchen 1 bis 8, dadurch gekennzeichnet, daß die Behandlung unter gleichzeitiger Desinfektion der Kieferabdrücke erfolgt.

10. Wässrige Desinfektionslösung für dentale Zwecke, enthaltend mindestens 0,5 Gew.-% eines Desinfektionsmittels, vorzugsweise aus der Gruppe der Aldehyde und/oder der organischen Persäuren sowie gegebenenfalls Alkohole, Tenside, Duft- und Farbstoffe, Antioxidationsmittel oder andere Hilfsmittel, dadurch gekennzeichnet, daß sie mindestens 0,01 bis 10 Gew.-% einer Fluorverbindung enthält.

11. Desinfektionslösung nach Anspruch 10, dadurch gekennzeichnet, daß sie 0,1 bis 5 Gew.-%, vorzugsweise 0,5 bis 2 Gew.-%, einer Fluorverbindung enthält.

12. Desinfektionslösung nach Anspruch 10 oder 11, dadurch gekennzeichnet, daß sie mindestens 0,1 Gew.-% eines Tensides enthält.

13. Desinfektionslösung nach Ansprüchen 10 bis 12, dadurch gekennzeichnet, daß sie als Fluorverbindung ein Metallfluorid, vorzugsweise aus der Gruppe bestehend aus den Alkalimetallfluoriden, Magnesiumfluorid, Zinkfluorid und Zinnfluoriden, enthält.

14. Desinfektionslösung nach Ansprüchen 10 bis 12, dadurch gekennzeichnet, daß sie als Fluorverbindung eine komplexe Fluorverbindung enthält.

15. Desinfektionslösung nach Anspruch 14, dadurch gekennzeichnet, daß sie eine Verbindung der Formel A₂MF₆ enthält, worin A ein Alkalimetall, vorzugsweise Natrium oder Kalium, und M Titan, Zirkonium oder Hafnium bedeuten.

16. Desinfektionslösung nach Anspruch 15, dadurch gekennzeichnet, daß sie K₂TiF₆ oder K₂ZrF₆ enthält.

17. Desinfektionslösung nach den Ansprüchen 10 bis 16, dadurch gekennzeichnet, daß sie 0,1 bis 10 Gew.-% eines mit Wasser mischbaren Alkohols enthält.

18. Verwendung der Desinfektionslösung nach den Ansprüchen 10 bis 17 zur Behandlung von Kieferabdrücken.

## Claims

1. Process for the treatment of jaw impressions composed of customary casting material, characterised in that the surface of the impressions is treated, before being filled with gypsum, with an aqueous solution containing 0.01 to 10% by wt. of a fluorine compound.

2. Process according to claim 1, characterised in that the solution contains 0.1 to 5% by wt., preferably 0.5 to 2% by wt. of a fluorine compound.

3. Process according to claims 1 or 2, characterised in that a metal fluoride, preferably from the group consisting of the alkali metal fluorides, magnesium fluoride, zinc fluoride and tin fluorides is used as fluorine compound.

4. Process according to claims 1 or 2, characterised in that a complex fluorine compound is used as fluorine compound.

5. Process according to claim 4, characterised in that a compound having the formula A₂MF₆ is used, wherein A means an alkali metal, preferably sodium or potassium, and M means titanium, zirconium or hafnium.

6. Process according to claim 5, characterised in that K₂TiF₆ or K₂ZrF₆ are used.

7. Process according to claims 1 to 6, characterised in that the solution further contains customary additives.

8. Process according to claim 7, characterised in that the treatment takes place by spraying or immersing the jaw impressions.

9. Process according to claims 1 to 8, characterised in that the treatment takes place with simultaneous disinfection of the jaw impressions.

10. Aqueous disinfection solution for dental purposes, containing at least 0.5% by wt. of a disinfectant, preferably from the group of aldehydes and/or organic peracids and optionally alcohols, surfactants, fragrances and dyes, antioxidants or other auxiliaries, characterised in that it contains at least 0.01 to 10% by wt. of a fluorine compound.

11. Disinfection solution according to claim 10, characterised in that it contains 0.1 to 5% by wt., preferably 0.5 to 2% by wt. of a fluorine compound.

12. Disinfection solution according to claim 10 or 11, characterised in that it contains at least 0.1% by wt. of a surfactant.

13. Disinfection solution according to claims 10 to 12, characterised in that it contains as fluorine compound a metal fluoride, preferably from the group consisting of the alkali metal fluorides, magnesium fluoride, zinc fluoride and tin fluorides.

14. Disinfection solution according to claims 10 to 12, characterised in that it contains as fluorine compound a complex fluorine compound.

15. Disinfection solution according to claim 14, characterised in that it contains a compound having the formula A₂MF₆, wherein A means an alkali metal, preferably sodium or potassium, and M means titanium, zirconium or hafnium.

16. Disinfection solution according to claim 15, characterised in that it contains K₂TiF₆ or K₂ZrF₆.

17. Disinfection solution according to claims 10 to 16, characterised in that it contains 0.1 to 10% by wt. of an alcohol miscible with water.

18. Use of the disinfection solution according to claims 10 to 17 for the treatment of jaw impressions.

## Revendications

1. Procédé de traitement d'empreintes de mâchoire constituées d'une matière usuelle de moulage, caractérisé en ce qu'avant d'y couler du plâtre, on traite la surface des empreintes à l'aide d'une solution aqueuse qui contient 0,01 à 10 % en poids d'un composé à base de fluor.

2. Procédé suivant la revendication 1, caractérisé en ce que la solution contient 0,1 à 5 % en poids, de préférence 0,5 à 2 % en poids, d'un composé à base de fluor.

3. Procédé suivant l'une des revendications 1 et 2, caractérisé en ce que, comme composé à base de fluor, on utilise un fluorure métallique, de préférence appartenant au groupe constitué des fluorures de métaux alcalins, du fluorure de magnésium, du fluorure de zinc et des fluorures d'étain.

4. Procédé suivant l'une des revendications 1 et 2, caractérisé en ce que, comme composé à base de fluor, on utilise un composé complexe à base de fluor.

5. Procédé suivant la revendication 4, caractérisé en ce qu'on utilise un composé de formule A₂MF₆_{,} dans laquelle A désigne un métal alcalin, de préférence le sodium ou le potassium, et M désigne le titane, le zirconium ou l'hafnium.

6. Procédé suivant la revendication 5, caractérisé en ce qu'on utilise K₂TiF₆ ou K₂ZrF₆.

7. Procédé suivant l'une des revendications 1 à 6, caractérisé en ce que la solution contient en outre des additifs usuels.

8. Procédé suivant la revendication 7, caractérisé en ce que le traitement s'effectue par pulvérisation ou immersion des empreintes de mâchoire.

9. Procédé suivant l'une des revendications 1 à 8, caractérisé en ce que le traitement s'effectue en même temps qu'une désinfection des empreintes de mâchoire.

10. Solution désinfectante aqueuse à usage dentaire, contenant au moins 0,5 % en poids d'un agent désinfectant, appartenant de préférence au groupe des aldéhydes et/ou des péracides organiques, ainsi qu'éventuellement des alcools, des agents tensio-actifs, des substances odoriférantes et des matières colorantes, des agents antioxydants ou d'autres agents auxiliaires, caractérisée en ce qu'elle contient au moins 0,01 à 10 % en poids d'un composé à base de fluor.

11. Solution désinfectante suivant la revendication 10, caractérisée en ce qu'elle contient 0,1 à 5 % en poids, de préférence 0,5 à 2 % en poids, d'un composé à base de fluor.

12. Solution désinfectante suivant l'une des revendications 10 et 11, caractérisée en ce qu'elle contient au moins 0,1 % en poids d'un agent tensio-actif.

13. Solution désinfectante suivant l'une des revendications 10 à 12, caractérisée en ce qu'elle contient, comme composé à base de fluor, un fluorure métallique, de préférence appartenant au groupe constitué des fluorures de métaux alcalins, du fluorure de magnésium, du fluorure de zinc et des fluorures d'étain.

14. Solution désinfectante suivant l'une des revendications 10 à 12, caractérisée en ce qu'elle contient, comme composé à base de fluor, un composé complexe à base de fluor.

15. Solution désinfectante suivant la revendication 14, caractérisée en ce qu'elle contient un composé de formule A₂MF₆_{,} dans laquelle A désigne un métal alcalin, de préférence le sodium ou le potassium, et M désigne le titane, le zirconium ou l'hafnium.

16. Solution désinfectante suivant la revendication 15, caractérisée en ce qu'elle contient K₂TiF₆ ou K₂ZrF₆.

17. Solution désinfectante suivant l'une des revendications 10 à 16, caractérisée en ce qu'elle contient 0,1 à 10 % en poids d'un alcool miscible à l'eau.

18. Application d'une solution désinfectante suivant l'une des revendications 10 à 17, au traitement d'empreintes de mâchoire.
